# EUROPEAN PATENT SPECIFICATION

(11) **EP 1 896 383 B1**
(45) Date of publication and mention of the grant of the patent: **09.09.2009**
(21) Application number: 06751916.5
(22) Date of filing: 28.04.2006
(51) Int. Cl.: C07C 5/48, C07C 11/04, C07C 51/215, C07C 53/08

(54) **METHOD FOR SELECTIVELY OXIDIZING ETHANE TO ETHYLENE**
VERFAHEN ZUR SELEKTIVEN OXIDIERUNG VON ETHAN ZU ETHYLEN
PROCEDE D'OXYDATION SELECTIVE DE L'ETHANE EN ETHYLENE

(30) Priority: 01.06.2005 US 686099 P
(43) Date of publication of application: 12.03.2008
(73) Proprietor: Celanese International Corporation, Dallas, TX 75234 (US)
(72) Inventor: RYAN, Debra, A., League City, Texas 77573-3077 (US)
(74) Representative: Kador & Partner
(86) International application number: PCT/US2006/016458
(87) International publication number: WO 2006/130288

(56) References cited:
- EP-A- 0 294 845
- US-A- 4 250 346
- US-A- 6 013 597
- USHIKUBO, OSHIMA, KAYOU, VAARKAMP, HATANO: "Ammoxidation of propane over catalysts comprising mixed oxides of Mo and V" JOURNAL OF CATALYSIS, vol. 169, 1997, pages 394-396, XP002396889
- LOPEZ NIETO J M ET AL: "THE SELECTIVE OXIDATIVE DEHYDROGENATION OF ETHANE OVER HYDROTHERMALLY SYNTHESISED MOVTENB CATALYSTS" CHEMICAL COMMUNICATIONS - CHEMCOM, ROYAL SOCIETY OF CHEMISTRY, GB, no. 17, 2002, pages 1906-1907, XP001157397 ISSN: 1359-7345

## Description

The invention relates to the production of ethylene. In particular, a method of selectively oxidizing ethane to ethylene using a mixed oxide catalyst containing vanadium and tungsten or molybdenum is disclosed.

### BACKGROUND OF THE INVENTION

The oxidative dehydrogenation of ethane to ethylene in the gas phase at temperatures above 500°C has been discussed, for example, in U.S. Patent Nos. 4,250,346, 4,524,236, and 4,568,790.

U.S. Patent No. 4,250,346 describes the use of a catalyst composition containing the elements molybdenum, X and Y in the ratio a:b:c for oxidation of ethane to ethylene, where X is Cr, Mn, Nb, Ta, Ti, V and/or W, and Y is Bi, Ce, Co, Cu, Fe, K, Mg, Ni, P, Pb, Sb, Si, Sn, Tl and/or U, and a is 1, b is from 0.05 to 1, and c is from 0 to 2. The total value of c for Co, Ni and/or Fe must be less than 0.5. The reaction is carried out in the gas phase at temperature below about 550°C. The efficiency of the conversion to ethylene ranges from 50 to 94%, depending upon ethane conversion. The catalysts disclosed can likewise be used for the oxidation of ethane to acetic acid, the efficiency of the conversion to acetic acid being about 18%, with an ethane conversion of 7.5%. Reaction pressures are very low, generally 1 atm, which restricts productivity and commercial viability.

U.S. Patent No. 4,568,790 describes a process for oxidizing ethane to ethylene using an oxide catalyst containing Mo, V, Nb, and Sb. The reaction is preferably carried out at about 200°C to about 450°C. The calculated selectivity for ethylene at 50% conversion of ethane ranges from 63 to 76%. Again low reaction pressures limit usefulness.

U.S. Patent No. 4,524,236 describes a process for oxidizing ethane to ethylene using an oxide catalyst containing Mo, V, Nb, and Sb and at least one metal from the group consisting of Li, Sc, Na, Be, Mg, Ca, Sr, Ba, Ti, Zr, Hf, Y, Ta, Cr, Fe, Co, Ni, Ce, La, Zn, Cd, Hg, Al, Tl, Pb, As, Bi, Te, U, and W. The reaction is preferably carried out at 200°C to about 400°C. The selectivity for ethylene at 51 % conversion of ethane is as high as 80% for one of the compositions discussed in the '236 patent, but productivity is low.

The above-mentioned specifications are principally concerned with the preparation of ethylene. The use of mixed metal oxide catalysts to convert ethane to acetic acid is also known. For example, U.S. Patent No. 5,162,578 describes a process for the selective preparation of acetic acid from ethane, ethylene or mixtures thereof with oxygen in the presence of a catalyst mixture which comprises at least: (A) a calcined catalyst of the formula MoₓV_{y} or MoₓV_{y}Z_{y}, in which Z can be one or more of the metals Li, Na, Be, Mg, Ca, Sr, Ba, Zn, Cd, Hg, Sc, Y, La, Ce, Al, Tl, Ti, Zr, Hf, Pb, Nb, Ta, As, Sb, Bi, Cr, W, U, Te, Fe, Co and Ni, and x is from 0.5 to 0.9, y is from 0.1 to 0.4, and z is from 0.001 to 1, and (B) an ethylene hydration catalyst and/or ethylene oxidation catalyst. The second catalyst component B is, in particular, a molecular sieve catalyst or a palladium-containing oxidation catalyst. The catalyst mixture was used to produce acetic acid and ethylene from a feed gas mixture consisting of ethane, oxygen, nitrogen and steam. The acetic selectivity was 34% and the ethylene selectivity was 62% with an ethane conversion of 4%. The high conversion rates of ethane were only achieved with the catalyst mixture described, but not in a single catalyst comprising components A and B.

A further process for the preparation of a product comprising ethylene and/or acetic acid is described in European Patent No. EP 0 407 091 B1. According to this process, ethane and/or ethylene and a gas containing molecular oxygen is brought into contact at elevated temperature with a mixed metal oxide catalyst composition of the general formula AₐX_{b}Y_{c} in which A is Mo_{d}ReₑW_{f}; X is Cr, Mn, Nb, Ta, Ti, V and/or W; Y is Bi, Ce, Co, Cu, Fe, K, Mg, Ni, P, Pb, Sb, Si, Sn, Tl and/or U; a is 1; b and c are independently 0 to 2; d+e+f=a, and e is nonzero. The selectivity for acetic acid or ethylene could be adjusted by adjusting the ratio of Mo to Re. The maximum selectivity obtained for acetic acid was 78% at 14.3% ethane conversion. The highest selectivity for ethylene was 70% at 15% ethane conversion.

It is therefore an object of the invention to provide a process that allows ethane and/or ethylene to be oxidized to ethylene in a simple and targeted manner and at high selectivity and space-time yield under reaction conditions which are as mild as possible.

### SUMMARY OF THE INVENTION

It has surprisingly been found that it is possible to oxidize ethane to ethylene under relatively mild conditions in a simple manner at high selectivity and excellent space-time yields when using a catalyst having the formula MoₐVᵥTaₓTe_{y}. a is 1.0; v is 0.01 to 1.0, more preferably 0.1 to 0.5; x is 0.01 to 1.0, more preferably 0.05 to 0.2; and y is 0.01 to 1.0, more preferably 0.1 to 0.5.

A further aspect of the invention provides a catalyst particularly suited for oxidizing ethane to produce ethylene. According to the particularly preferred embodiment, the catalyst has the formula Mo_{1.0}V_{0.3}Ta_{0.1}Te_{0.3}O_{z}, where z depends on the oxidation state of the metals and is the number that renders the catalyst electronically neutral.

### DETAILED DESCRIPTION OF THE INVENTION

The present invention provides a process for selectively preparing ethylene from a gaseous feed comprising ethane and oxygen, by bringing the gaseous feed into contact with catalyst haying the formula MoₐVᵥTaₓTe_{y}. a is 1.0; v is 0.01 to 1.0, more preferably 0.1 to 0.5; x is 0.01 to 1.0, more preferably 0.05 to 0.2; and y is 0.01 to 1.0, more preferably 0.1 to 0.5. As used herein, the catalyst is referred to using the formula MoₐVᵥTaₓTe_{y}. One of skill in the art will appreciate that the catalyst is actually a mixed oxide having the formula MoₐVᵥTaₓTe_{y} O_{z}. The amount of oxygen, z, is determined by the oxidation states of Mo, V, Ta, and Te and cannot be generally specified.

According to a preferred embodiment, the catalyst has the formula MoₐVᵥTaₓTe_{y}O_{z} wherein a, v, x, and y have the ranges specified above. A particularly preferred catalyst has the formula Mo_{1.0}V_{0.3}Ta_{0.1}Te_{0.3}O_{z}.

The catalyst of the invention can be prepared as described in U.S. Patent No. 6,653,253, by Lin, the entire contents of which are incorporated herein by reference. Briefly, metal compounds that are the sources of the metals in the catalyst are combined in at least one solvent in appropriate amounts to form a solution. Generally, the metal compounds contain elements Mo, V, Ta, Te, and at least one of the metal compounds contains O. For example, a compound according to AₐVᵥXₓY_{y}O wherein A is Mo, X is Ta, and Y is Te, can be prepared by combining an aqueous solution of tantalum oxalate with an aqueous solution or slurry of ammonium heptamolybdate, ammonium metavanadate and telluric acid, wherein the concentrations of the metal compounds are such that the atomic ratio of the respective metal elements are in the proportions prescribed by the stoichiometry of the target catalyst

Additionally, a wide range of materials including, oxides, nitrates, halides or oxyhalides, alkoxides, acetylacetonates, and organometallic compounds may be used. For example, ammonium heptamolybdate may be used as the source of molybdenum in the catalyst However, compounds such as MoO₃, MoO₂ Mods, MoOCl₄, Mo(OC₂H₅)₅, molybdenum acetylacetonate, phosphomolybdic acid and silicomolybdic acid may also be utilized instead of ammonium heptamolybdate. Similarly, ammonium metavanadate may be used as the source of vanadium in the catalyst However, compounds such as V₂O₅, V₂O₃, VOCl₃, VCl₄, VO(OC₂H₅), vanadium acetylacetonate and vanadyl acetylacetonate may also be utilized instead of ammonium metavanadate. The tellurium source may include telluric acid, TeCl₄, Te(OC₂H₅)₅, Te(OCH(CH₃)₂)₄ and TeO₂. The tantalum source may include ammonium tantalum oxalate, Ta₂O₅, TaCl₅, tantalic acid or Ta(OC₂H₅)₅ as well as the more conventional tantalum oxalate.

Suitable solvents include water, alcohols (including but not limited to methanol, ethanol, propanol, and diols etc.) as well as other polar solvents known in the art. Generally, water is preferred. The water is any water suitable for use in chemical synthesis including, without limitation, distilled water and deionized water. The amount of water present is that amount sufficient to keep the elements substantially in solution long enough to avoid or minimize compositional and/or phase segregation during the preparation steps. Once the aqueous solution is formed, the water is removed by a combination of any suitable methods known in the art to form a catalyst precursor. Such methods include, without limitation, vacuum drying, freeze drying, spray drying, rotary evaporation, and air drying. Rotary evaporation or air drying are generally preferred.

Once obtained, the catalyst precursor is calcined under an inert atmosphere. The inert atmosphere may be any material which is substantially inert to, i.e., does not react or interact with, the catalyst precursor. Suitable examples include, without limitation, nitrogen, argon, xenon, helium or mixtures thereof. Preferably, the inert atmosphere is argon or nitrogen, more preferably argon. The inert atmosphere may or may not flow over the surface of the catalyst precursor. Typically, if nitrogen is used, flowing is used. If the inert atmosphere is argon, then typically flowing is not used. When the inert atmosphere does flow over the surface of the catalyst precursor, the flow rate can vary over a wide range, for example, at a space velocity from 1 to 500 hr⁻¹. The calcination is typically done at a temperature of from 350°C to 850°C, preferably from 400°C to 700°C, more preferably from 500°C to 640°C. The calcination is performed for long enough to form the catalyst. In one embodiment, the calcination is performed from 0.5 to 30 hours, preferably from 1 to 25 hours and more preferably from 1 to 15 hours.

The catalyst of the invention may be used as a solid catalyst alone or may be used with a suitable support. Conventional support materials are suitable, for example, porous silicon dioxide, ignited silicon dioxide, kieselguhr, silica gel, porous or nonporous aluminum oxide, titanium dioxide, zirconium dioxide, thorium dioxide, lanthanum oxide, magnesium oxide, calcium oxide, barium oxide, tin oxide, cerium dioxide, zinc oxide, boron oxide, boron nitride, boron carbide, boron phosphate, zirconium phosphate, aluminum silicate, silicon nitride or silicon carbide, but also glass, carbon-fiber, carbon, activated carbon, metal-oxide or metal networks or corresponding monoliths.

Support materials should be chosen based on optimizing both the surface area and pore size for the specific oxidation of interest. The catalyst can be employed after shaping as a regularly or irregularly shaped support element, but also in powder form as a heterogeneous oxidation catalyst.

Alternatively, the catalyst of the invention may be encapsulated in a material. Suitable materials for encapsulation include SiO₂, P₂O₅, MgO, Cr₂O₃, TiO₂, ZrO₂, and Al₂O₃. Methods of encapsulating materials in oxides are known in the art. A suitable method of encapsulating materials in oxides is described in U.S. Patent No. 4,677,084 and references cited therein, the entire contents of which are incorporated herein by references.

The oxidation of ethane can be carried out in a fluidized bed or in a fixed bed reactor. For use in a fluidized bed, the catalyst is normally ground to a particle size in the range from 10 to 200 µm or prepared by spray drying.

The gaseous feedstock, and any recycle gas combined with said feedstock gas, contains primarily ethane but may contain some amount of ethylene, and is fed to the reactor as a pure gas or in a mixture with one or more other gases. Suitable examples of such additional or carrier gases are nitrogen, methane, carbon monoxide, carbon dioxide, air and/or steam. The gas containing molecular oxygen may be air or a gas which has a higher or lower molecular oxygen concentration than air, for example pure oxygen.

The reaction is generally carried out at 200 to 500°C, preferably 200 to 400°C. The pressure can be atmospheric or superatmospheric, for example about 1 to 50 bar, preferably 1 to 30 bar.

The reaction can be carried out in a fixed bed or fluidized bed reactor. Ethane can be first mixed with an inert gas such as nitrogen or steam before oxygen or the gas containing molecular oxygen is fed in. The mixed gases can be preheated to the reaction temperature in a preheating zone before the gas mixture is brought into contact with the catalyst. Acetic acid can be removed from the gas leaving the reactor by condensation. The other gases can be returned to the reactor inlet, where oxygen or the gas containing molecular oxygen, and ethane is metered in.

According to a preferred embodiment, ethane feed is purified and distilled to provide purified ethane as a top stream and propane and other heavies as a bottom stream. The ethane is provided to an oxidation reactor, which is a fluidized bed reactor utilizing the catalyst described above. According to a particularly preferred embodiment, the catalyst has the formula MoₐVᵥTaₓTe_{y}O_{z}, where a, v, x, y, and z are as defined above. According to an especially preferred embodiment, the catalyst has the formula Mo_{1.0}V_{0.3}Ta_{0.1}Te_{0.3}O_{z}. Oxygen is also provided to the reactor.

The oxidation reaction produces a mixture of gases including ethylene, acetic acid, water, COₓ (CO and CO₂), unreacted ethane, and assorted heavy by-products. The product gas effluent from the reactor is preferably filtered to remove catalyst fines and is then routed to a recycle gas scrubber, which produces a top stream containing ethylene, ethane, and COₓ. The top stream from the recycle gas scrubber is routed to a fixed bed CO converter followed by a processing step that removes the COₓ from the top stream. The stream is then routed to an ethylene purification tower that provides product ethylene as a top stream and ethane as a bottom stream, which is recycled to the oxidation reactor.

The bottom stream from the recycle gas scrubber, which contains acetic acid, water, and heavy ends by-products, may be purified as known in the art to provide purified acetic acid. For example, the bottom stream may be routed to a drying column to remove water followed by a heavy ends column to remove propionic acid and other heavy components.

One of skill in the art will appreciate that the towers, scrubbers, and routing referred to in the preceding paragraphs will have associated with them various heat exchangers, pumps, and connectors and will have operating parameters that are determined by the particular mixture of gases involved. It is within the ability of one of ordinary skill in the art to determine the proper configurations and parameters, given the above disclosure.

A further aspect of the invention is a catalyst that is particularly suitable for the oxidation of ethane to produce ethylene and acetic acid with a high selectivity for ethylene. Preferably, the selectivity for ethylene is 80%, more preferably 50% to 80%, even more preferably 70% to 80%. According to a preferred embodiment, the catalyst has the formula MoₐVᵥTaₓTe_{y}O_{z}, where a, v, x, y, and z are as defined above. According to particularly preferred embodiment, the catalyst has the formula Mo_{1.0}V_{0.33}Ta_{0.12}Te_{0.28}O_{z}.

The following examples are included to demonstrate preferred embodiments of the invention. It should be appreciated by those of skill in the art that the techniques disclosed in the following examples represent techniques discovered by the inventors to function well in the practice of the invention, and thus can be considered to constitute preferred modes for its practice. However, those of skill in the art should appreciate, in light of the present disclosure, that many changes can be made in the specific embodiments which are disclosed and still obtain a like or similar result without departing from the scope of the invention.

### EXAMPLE

### Example 1:

A catalyst having the formula Mo₁V_{0.33}Ta_{0.12}Te_{0.28}O_{z} is prepared as follows: 25.0 g of ammonium heptamolybdate tetrahydrate (Aldrich Chemical Company), 5.47 g of ammonium metavanadate (Aldrich Chemical Company) and 9.10 g of telluric acid (Aldrich Chemical Company) are dissolved in 400 mL of water by heating to 80°C. After cooling to room temperature, 28.0 mL of an aqueous solution of tantalum oxalate (0.5 M Ta, 1.5 M oxalate) is added. The water is removed via a rotary evaporator with a warm water bath at 50°C to obtain the catalyst precursor solid. The solid is dried at 120 C prior to calcination.

The catalyst precursor solid is calcined under a nitrogen atmosphere in a covered crucible pre-purged with nitrogen 600°C for 2 hours. The oven is ramped at 10 deg C/min to %0 C and held for 2 hours, and then reampned to 600 C at 10 C/min, and held at 600 C for 2 hours. The catalyst thus obtained is ground to a fine powder and pressed in a mold and then broken and sieved to 600-710 micron particles.

About 3 mL of the catalyst was mixed with about 7 mL of quartz particles and loaded into the bottom half of a stainless steel tube reactor with an internal diameter of 7.7 mm. Quartz is layered onto the top of the catalyst bed to both fill the reactor and to preheat the gaseous feeds prior to entering the catalyst bed. The reactor is heated and cooled by use of thermostated oil circulating in an external jacket. Water is vaporized in an evaporator and mixed with the desired volumes of ethane, oxygen, and nitrogen gases before being supplied to the reactor through mass flow controllers. The reaction pressure is maintained at the desired value by a back pressure regulator located on the reactor vent gas. The temperature in the catalyst bed is measured by a moveable thermocouple inserted in a thermowell in the center of the catalyst bed. The temperature is increased in the oil jacket until the desired oxygen conversion is achieved. The reaction feed gas and the product gas are analyzed on-line by gas chromatography.

The contact time is defined as:
- t (sec) =: bulk volume of the catalyst (mL)/a volume flow rate of the feed gas through the reactor at reaction conditions (mL/s).
- GHSV =: the gas hourly space velocity, is the reciprocal of the contact time, t, corrected to STP (0 °C, 1 atm).

The ethane concentration in the feed was varied from 37 to 67 mol%, the oxygen concentration in the feed was varied from 7.6 to 15.3 mol%, and the water was varied from 4 to 9 mol%, with the balance being made up with nitrogen, as shown in Table 1. A very high selectivity to ethylene of 74 to 80% is achieved over a range of contact times, as shown in Table 2. Additionally, the selectivity to CO₂ and CO is very low, the sum never more than 8% over the range of conditions tested. Productivity as measured by the STY to ethylene is likewise very high with values as high as 460 kg ethylene per m³ per hour.

**Table 1: Reaction Conditions**

| | Reaction Conditions | | | | | | | | | |
|---|---|---|---|---|---|---|---|---|---|---|
| Sample | Ethane (%) | Ethylene (%) | Oxygen (%) | Nitrogen (%) | Water (%) | P MPa (psig) | T (sec) | GHSV (hr⁻¹) | T,Center | T,Shell |
| 1 | **39** | **0** | **8.1** | **43** | **5** | 1.52 **(220)** | **10.2** | **2561** | **328** | **na** |
| 2 | **38** | **0** | **7.5** | **40** | **11** | 1.52 **(220)** | **9.5** | **2732** | **318** | **na** |
| 3 | **37** | **0** | **8.4** | **49** | **9** | 1.49 **(216)** | **9.7** | **2743** | **309** | **308** |
| 4 | **39** | **0** | **8.6** | **50** | **7** | 1.50 **(218)** | **9.7** | **2746** | **309** | **308** |
| 5 | **38** | **0** | **8.7** | **53** | **3** | 1.50 **(217)** | **9.6** | **2743** | **314** | **315** |
| 6 | **46** | **0** | **14.9** | **54** | **7** | 1.49 **(216)** | **9.4** | **2738** | **323** | **320** |
| 7 | **38** | **0** | **153** | **41** | **5** | 1.48 **(215)** | **9.3** | **2732** | **332** | **327** |
| 8 | **38** | **0** | **12.6** | **44** | **5** | 1.48 **(215)** | **9.5** | **2742** | **320** | **319** |
| 9 | **40** | **0** | **14.4** | **41** | **4** | 1.48 **(215)** | **14.4** | **1808** | **318** | **315** |
| 10 | **54** | **0** | **7.6** | **33** | **5** | 1.50 **(217)** | **9.8** | **2740** | **305** | **303** |
| 11 | **66** | **0** | **7.8** | **19** | 5 | 1.50 **(217)** | **10.0** | **2739** | **295** | **303** |
| 12 | **66** | **0** | **12.0** | **14** | **5** | 1.49 **(216)** | **9.6** | **2737** | **315** | **312** |
| 13 | **65** | **0** | **15.1** | **11** | **5** | 1.48 **(215)** | **9.4** | **2737** | **322** | **317** |
| 14 | **67** | **0** | **7.7** | **17** | **5** | 1.49 **(216)** | **15.1** | **1814** | **290** | **291** |
| 15 | **67** | **0** | **12** | **13.6** | **4** | 1.49 **(216)** | **14.8** | **1814** | **303** | **301** |
| 16 | **67** | **15** | **15** | **10.9** | **4** | 1.48 **(215)** | **14.6** | **1813** | **310** | **307** |
| 17 | **66** | **14** | **14** | **15.5** | **0** | 1.49 **(216)** | **14.4** | **1826** | **312** | **na** |

**Table 2: Catalyst Performance**

| Sample | Ethane Conv. (%) | O2 Conv (%). | Ethylene Sel (%) | CO₂ Sel (%) | CO Sel (%) | STY,Ethylene |
|---|---|---|---|---|---|---|
| 1 | **24** | **91** | **79** | **1** | **3** | **258** |
| 2 | **23** | **95** | **75** | **1** | **3** | **241** |
| 3 | **24** | **93** | **77** | **1** | **3** | **252** |
| 4 | **24** | **93** | **76** | **1** | **3** | **247** |
| 5 | **25** | **94** | **80** | **1** | **3** | **276** |
| 6 | **32** | **86** | **79** | **2** | **3** | **344** |
| 7 | **42** | **94** | **76** | **3** | **5** | **436** |
| 8 | **32** | **93** | **77** | **2** | **4** | **354** |
| 9 | **39** | **96** | **74** | **2** | **5** | **261** |
| 10 | **16** | **88** | **77** | **1** | **2** | **236** |
| 11 | **14** | **95** | **78** | **1** | **2** | **265** |
| 12 | **21** | **97** | **76** | **1** | **3** | **382** |
| 13 | **26** | **97** | **75** | **2** | **4** | **460** |
| 14 | **13** | **92** | **77** | **1** | **2** | **160** |
| 15 | **19** | **98** | **74** | **1** | **3** | **230** |
| 16 | **24** | **98** | **73** | **2** | **4** | **274** |
| 17 | **24** | **97** | **77** | **2** | **4** | **298** |

These results are a significant improvement compared to prior art. For example, the catalyst Mo ₂.₅ V₁Nb _{0.32} Te ₁._{69E-05} described in Example 10 of US 6013957 produced only a 28.4% selectivity to ethylene, and while the selectivities to CO2 and CO were not reported, if it is assumed that the products not reported are indeed COx, then this inefficiency could be as high as 34.4%. Likewise, Example B of WO 2004/108277 reported only a 5% selectivity to ethylene for catalyst Mo₁V ₀.₅₂₉ Nb ₀.₁₂₄ Ti ₀.₃₃₁. with 35% selectivity to COₓ. So the present catalyst offers high selectivity to ethylene with much lower loss to the deep oxdiation products, COₓ.

## Claims

1. A process for preparing ethylene from a gaseous feed comprising ethane and oxygen, said process comprising contacting the gaseous feed with a catalyst in a reactor to produce an effluent comprising ethylene, the catalyst having the formula
MoₐVᵥTaₓTe_{y}O_{z}
wherein, a is 1.0, v is 0.01 to 1.0, x is 0.01 to 1.0, and y is 0.01 to 1.0, and z is the number of oxygen atoms necessary to render the catalyst electronically neutral.

2. The process of claim **1,** wherein the gaseous feed further comprises ethylene.

3. The process of claim **1,** wherein a is 1.0, v is 0.1 to 0.5, x is 0.05 to 0.2, and y is 0.1 to 0.5.

4. The process of claim **1,** wherein the catalyst has the formula Mo_{1.0}V_{0.3}Ta_{0.1}Te_{0.3}O_{z}.

5. The process of claim **1,** wherein the reactor is a fixed bed reactor containing the catalyst.

6. The process of claim **1,** wherein the reactor is a fluidized bed reactor containing the catalyst.

7. The process of claim **1,** wherein the gaseous feed contacts the catalyst at a temperature of 200°C to 500°C.

8. The process of claim **7**, wherein the gaseous feed contacts the catalyst at a temperature of 200°C to 400°C.

9. The process of claim **1**, wherein the catalyst is supported on a support selected from the group consisting of porous silicon dioxide, ignited silicon dioxide, kieselguhr, silica gel, porous and nonporous aluminum oxide, titanium dioxide, zirconium dioxide, thorium dioxide, lanthanum oxide, magnesium oxide, calcium oxide, barium oxide, tin oxide, cerium dioxide, zinc oxide, boron oxide, boron nitride, boron carbide, boron phosphate, zirconium phosphate, aluminum silicate, silicon nitride, silicon carbide, and glass, carbon, carbon-fiber, activated carbon, metal-oxide or metal networks and corresponding monoliths.

10. The process of claim **1**, wherein the catalyst is not supported on a support.

11. The process of claim **1**, wherein the catalyst is encapsulated in a material.

12. The process of claim **11**, wherein the material is selected from the group consisting of SiO₂, P₂O₅, MgO, Cr₂O₃, TiO₂, ZrO₂, and Al₂O₃.

13. The process of claim **1**, further comprising the step of separating a feed precursor comprising ethane and propane to provide the ethane.

14. The process of claim **1**, wherein the effluent comprises carbon monoxide, further comprising the step of selectively oxidizing said effluent to convert the carbon monoxide to carbon dioxide.

15. The process of claim **14**, further comprising the step of removing the carbon dioxide from the effluent.

16. The process of any one of claims **1, 13**, or **15**, further comprising the step of distilling the effluent to remove unreacted ethane therefrom.

17. The process of claim **16**, further comprising the step of recycling the unreacted ethane to the reactor.

18. The process of any one of claims **1**, **13**, or **15**, wherein the effluent comprises acetic acid, the process further comprising the step of separating the acetic acid from the effluent.

19. The process of claim **18**, wherein the effluent comprises water, propionic acid, or a mixture thereof, the process further comprising the step of separating said water and said propionic acid from the acetic acid.

20. The process of any one of claims **1, 13, 14**, or **15**, further comprising the step of reacting the ethylene with acetic acid to produce vinyl acetate.

21. The process of claim **20**, wherein at least some of the acetic acid is produced in the reactor.

22. A process for oxidizing ethane to produce ethylene and acetic acid, comprising contacting a catalyst with a gaseous feed comprising ethane and oxygen at a temperature of 200°C to 400°C, wherein the catalyst has the formula Mo_{1.0}V_{0.33}Ta_{0.12}Te_{0.28}O_{z}.

## Patentansprüche

1. Verfahren zum Herstellen von Ethylen aus einer gasförmigen Beschickung, die Ethan und Sauerstoff aufweist, wobei das Verfahren das Inkontaktbringen der gasförmigen Beschickung mit einem Katalysator in einem Reaktor umfaßt, so daß ein Abfluß erzeugt wird, der Ethylen aufweist, wobei der Katalysator die folgende Formel hat:
MoₐVᵥTaₓTe_{y}O_{z}
worin
a gleich 1,0 ist, v gleich 0,01 bis 1,0 ist, x gleich 0,01 bis 1,0 ist und y gleich 0,01 bis 1,0 ist und z die Anzahl der Sauerstoffatome ist, die erforderlich sind, um den Katalysator elektrisch neutral zu machen.

2. Verfahren nach Anspruch 1, wobei die gasförmige Beschickung ferner Ethylen aufweist.

3. Verfahren nach Anspruch 1, wobei a gleich 1,0 ist, v gleich 0,1 bis 0,5 ist, x gleich 0,05 bis 0,2 ist und y gleich 0,1 bis 0,5 ist.

4. Verfahren nach Anspruch 1, wobei der Katalysator die folgende Formel hat:
Mo_{1,0}V_{0,3}Ta_{0,1}Te_{0,3}O_{z}.

5. Verfahren nach Anspruch 1, wobei der Reaktor ein Festbettreaktor ist, der den Katalysator enthält.

6. Verfahren nach Anspruch 1, wobei der Reaktor ein Wirbelbettreaktor ist, der den Katalysator enthält.

7. Verfahren nach Anspruch 1, wobei die gasförmige Beschickung bei einer Temperatur von 200 bis 500°C mit dem Katalysator in Kontakt kommt.

8. Verfahren nach Anspruch 7, wobei die gasförmige Beschickung bei einer Temperatur von 200 bis 400°C mit dem Katalysator in Kontakt kommt.

9. Verfahren nach Anspruch 1, wobei der Katalysator auf einem Träger getragen wird, der aus der Gruppe ausgewählt ist, bestehend aus porösem Siliciumdioxid, geglühtem Siliciumdioxid, Kieselgur, Kieselgel, porösem und nichtporösem Aluminiumoxid, Titandioxid, Zirconiumdioxid, Thoriumdioxid, Lanthanoxid, Magnesiumoxid, Calciumoxid, Bariumoxid, Zinnoxid, Cerdioxid, Zinkoxid, Boroxid, Bornitrid, Borcarbid, Borphosphat, Zirconiumphosphat, Aluminiumsilicat, Siliciumnitrid, Siliciumcarbid und Glas, Kohle, Kohlenstoffasern, Aktivkohle, Metalloxid- oder Metallnetzen und entsprechenden Monolithen.

10. Verfahren nach Anspruch 1, wobei der Katalysator nicht auf einem Träger getragen wird.

11. Verfahren nach Anspruch 1, wobei der Katalysator in einem Material verkapselt ist.

12. Verfahren nach Anspruch 11, wobei das Material aus der Gruppe ausgewählt ist, bestehend aus SiO₂, P₂O₅ MgO, Cr₂O₃, TiO₂, ZrO₂ und Al₂O₃.

13. Verfahren nach Anspruch 1, das ferner einen Schritt umfaßt, bei dem eine Beschickungsvorstufe, die Ethan und Propan umfaßt, abgetrennt wird, so daß das Ethan bereitgestellt wird.

14. Verfahren nach Anspruch 1, bei dem der Abfluß Kohlenmonoxid umfaßt, das ferner einen Schritt aufweist, bei dem dieser Abfluß selektiv oxidiert wird, so daß das Kohlenmonoxid in Kohlendioxid überführt wird.

15. Verfahren nach Anspruch 14, das ferner einen Schritt umfaßt, bei dem das Kohlendioxid aus dem Abfluß entfernt wird.

16. Verfahren nach einem der Ansprüche 1, 13 oder 15, das ferner einen Schritt umfaßt, bei dem der Abfluß destilliert wird, so daß unreagiertes Ethan daraus entfernt wird.

17. Verfahren nach Anspruch 16, das ferner einen Schritt umfaßt, bei dem das unreagierte Ethan zum Reaktor rezirkuliert wird.

18. Verfahren nach einem der Ansprüche 1, 13 oder 15, bei dem der Abfluß Essigsäure umfaßt, wobei das Verfahren ferner einen Schritt umfaßt, bei dem die Essigsäure aus dem Abfluß abgetrennt wird.

19. Verfahren nach Anspruch 18, bei dem der Abfluß Wasser, Propionsäure oder ein Gemisch davon umfaßt, wobei das Verfahren ferner einen Schritt umfaßt, bei dem das Wasser und die Propionsäure von der Essigsäure abgetrennt werden.

20. Verfahren nach einem der Ansprüche 1, 13, 14 oder 15, das ferner einen Schritt umfaßt, bei dem das Ethylen mit Essigsäure umgesetzt wird, so daß Vinylacetat erzeugt wird.

21. Verfahren nach Anspruch 20, wobei zumindest ein Teil der Essigsäure im Reaktor erzeugt wird.

22. Verfahren zum Oxidieren von Ethan, so daß Ethylen und Essigsäure erzeugt werden, das das Inkontaktbringen eines Katalysators mit einer gasförmigen Beschickung, die Ethan und Sauerstoff umfaßt, bei einer Temperatur von 200 bis 400°C umfaßt, wobei der Katalysator die Formel Mo_{1,0}V_{0,33}Ta_{0,12}Te_{0,28}O_{z} hat.

## Revendications

1. Procédé pour la préparation d'éthylène à partir d'une charge gazeuse de départ, comprenant de l'éthane et de l'oxygène, ledit procédé comprenant la mise en contact de la charge gazeuse de départ avec un catalyseur dans un réacteur pour produire un effluent comprenant de l'éthylène, le catalyseur ayant la formule :
MoₐVᵥTaₓTe_{y}O_{z}
dans laquelle, a est égal à 1,0, v varie de 0,01 à 1,0, x varie de 0,01 à 1,0 et y varie de 0,01 à 1,0, et z est le nombre d'atomes d'oxygène nécessaires pour rendre le catalyseur électroniquement neutre.

2. Le procédé de la revendication 1, dans lequel la charge gazeuse de départ comprend, en outre, de l'éthylène.

3. Le procédé de la revendication 1, dans lequel a est égal à 1,0, v varie de 0,1 à 0,5, x varie de 0,05 à 0,2 et y varie de 0,1 à 0,5.

4. Le procédé de la revendication 1, dans lequel le catalyseur a la formule Mo_{1,0}V_{0,3}Ta_{0,1}Te_{0,3}O_{z}.

5. Le procédé de la revendication 1, dans lequel le réacteur est un réacteur à lit fixe, contenant le catalyseur.

6. Le procédé de la revendication 1, dans lequel le réacteur est un réacteur à lit fluidisé, contenant le catalyseur.

7. Le procédé de la revendication 1, dans lequel la charge gazeuse de départ vient au contact du catalyseur à une température de 200 °C à 500 °C.

8. Le procédé de la revendication 7, dans lequel la charge gazeuse de départ vient au contact du catalyseur à une température de 200 °C à 400 °C.

9. Le procédé de la revendication 1, dans lequel le catalyseur est supporté sur un support choisi parmi le groupe consistant en le dioxyde de silicium poreux, le dioxyde de silicium calciné, le kieselguhr, un gel de silice, les oxydes d'aluminium poreux et non poreux, le dioxyde de titane, le dioxyde de zirconium, le dioxyde de thorium, l'oxyde de lanthane, l'oxyde de magnésium, l'oxyde de calcium, l'oxyde de baryum, l'oxyde d'étain, le dioxyde de cérium, l'oxyde de zinc, l'oxyde de bore, le nitrure de bore, le carbure de bore, le phosphate de bore, le phosphate de zirconium, le silicate d'aluminium, le nitrure de silicium, le carbure de silicium, et le verre, le carbone, une fibre de carbone, le carbone activé, les réseaux d'oxyde de métal ou de métal, et les monolithes correspondants.

10. Le procédé de la revendication 1, dans lequel le catalyseur n'est pas supporté par un support.

11. Le procédé de la revendication 1, dans lequel le catalyseur est encapsulé dans une matière.

12. Le procédé de la revendication 11, dans lequel le matériau est choisi parmi le groupe consistant en SiO₂, P₂O₅, MgO, Cr₂O₃, TiO₂, ZrO₂ et Al₂O₃.

13. Le procédé de la revendication 1, comprenant, en outre, l'étape de séparation d'un précurseur de départ, comprenant de l'éthane et du propane, pour produire l'éthane.

14. Le procédé de la revendication 1, dans lequel l'effluent comprend du monoxyde de carbone, et comprenant, en outre, l'étape d'oxydation sélective du dit effluent pour convertir le monoxyde de carbone en dioxyde de carbone.

15. Le procédé de la revendication de 14, comprenant, en outre, l'étape d'élimination du dioxyde de carbone à partir de l'effluent.

16. Le procédé de l'une quelconque des revendications 1, 13 ou 15, comprenant en outre l'étape de distillation de l'effluent pour y retirer l'éthane n'ayant pas réagi.

17. Le procédé de la revendication 16, comprenant, en outre, l'étape de recyclage de l'éthane n'ayant pas réagi, dans le réacteur.

18. Le procédé de l'une quelconque des revendications 1, 13 ou 15, dans lequel l'effluent comprend de l'acide acétique, le procédé comprenant, en outre, l'étape de séparation de l'acide acétique à partir de l'effluent.

19. Le procédé de la revendication 18, dans lequel l'effluent comprend de l'eau, de l'acide propionique ou un mélange de ceux-ci, le procédé comprenant, en outre, l'étape de séparation de ladite eau et du dit acide propionique à partir de l'acide acétique.

20. Le procédé de l'une quelconque des revendications 1, 13, 14 ou 15, comprenant, en outre, l'étape de réaction de l'éthylène avec l'acide acétique pour produire de l'acétate de vinyle.

21. Le procédé de la revendication 20, dans lequel au moins une partie de l'acide acétique est produite dans le réacteur.

22. Procédé d'oxydation d'éthane pour produire de l'éthylène et de l'acide acétique, comprenant la mise en contact d'un catalyseur avec une charge gazeuse de départ, comprenant de l'éthane et de l'oxygène, à une température de 200 °C à 400 °C, dans lequel le catalyseur a la formule Mo_{1,0}V_{0,33}Ta_{0,12}Te ₀,₂₈O_{z}.
